# EUROPÄISCHE PATENTSCHRIFT

(11) **EP 1 418 915 B1**
(45) Veröffentlichungstag und Bekanntmachung des Hinweises auf die Patenterteilung: **10.01.2007**
(21) Anmeldenummer: 02794539.3
(22) Anmeldetag: 02.08.2002
(51) Int. Cl.: A61K 31/495, A61P 25/16, A61P 25/28

(54) **NEUROPROTEKTIVES MEDIKAMENT**
NEUROPROTECTIVE DRUG
MEDICAMENT NEUROPROTECTEUR

(30) Priorität: 10.08.2001 DE 10138273
(43) Veröffentlichungstag der Anmeldung: 19.05.2004
(60) Teilanmeldung: 06123673.3
(73) Patentinhaber: Boehringer Ingelheim Pharma GmbH & Co.KG, 55216 Ingelheim am Rhein (DE)
(72) Erfinder: BORSINI, Franco, 88339 BAD WALDSEE (DE)
(86) Internationale Anmeldenummer: PCT/EP2002/008614
(87) Internationale Veröffentlichungsnummer: WO 2003/013539

(56) Entgegenhaltungen:
- EP-A- 0 526 434
- EP-A- 0 982 030
- EP-A- 1 256 343
- WO-A-98/42344
- DARLINGTON: "Flibaserin" CURRENT OPINION IN CPNS INVESTIGATIONAL DRUGS, Bd. 1, Nr. 4, 1999, Seite 510-513 XP001119016

## Beschreibung

Die vorliegende Erfindung betrifft die Verwendung von 1-[2-(4-(3-Trifluoromethylphenyl)piperazin-1-yl)ethyl]-2,3-dihydro-1H-benzimidazol-2-on, gegebenenfalls in Form der pharmazeutisch verträglichen Säureadditionssalze sowie gegebenenfalls in Form der Hydrate oder Solvate zur Herstellung eines Arzneimittels mit neuroprotektiver Wirkung.

### Hintergrund der Erfindung

Die Verbindung 1-(2-(4-(3-Trifluoromethyl-phenyl)piperazin-1-yl)ethyl]-2,3-dihydro-1H-benzimidazol-2-on (Flibanserin) ist in Form ihres Hydrochlorids aus der europäischen Patentanmeldung EP-A-526434 bekannt und weist die folgende chemische Struktur auf:

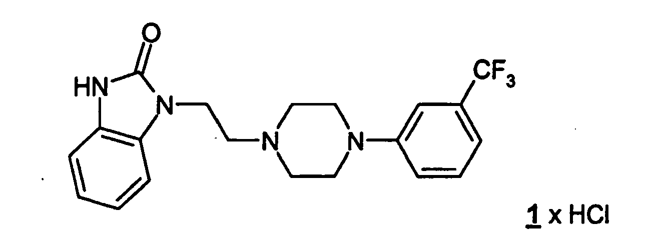

Flibanserin zeigt eine Affinität zum 5-HT_{1A} und 5-HT₂-Rezeptor. Aus diesem Grund ist es zur Behandlung einer Vielzahl von Erkrankungen therapeutisch einsetzbar. Hierzu zählen beispielsweise Depression, Schizophrenie, Parkinson, Angstzustände, oder auch beispielsweise Schlafstörungen.

### Beschreibung der Erfindung

Überraschenderweise wurde gefunden, daß die Verbindung 1-[2-(4-(3-Trifluoromethyl-phenyl)piperazin-1-yl)ethyl]-2,3-dihydro-1H-benzimidazol-2-on, gegebenenfalls in Form der pharmazeutisch verträglichen Säureadditionssalze sowie gegebenenfalls in Form der Hydrate oder Solvate ferner zur Herstellung eines Arzneimittels mit neuroprotektiver Wirkung Verwendung finden kann.

Dementsprechend betrifft die vorliegende Erfindung die Verwendung von 1-[2-(4-(3-Trifluoromethyl-phenyl)piperazin-1-yl)ethyl]-2,3-dihydro-1 H-benzimidazol-2-on, gegebenenfalls in Form der pharmazeutisch verträglichen Säureadditionssalze sowie gegebenenfalls in Form der Hydrate oder Solvate zur Herstellung eines Arzneimittels mit neuroprotektiver Wirkung.

Bevorzugt betrifft die vorliegende Erfindung die Verwendung von 1-[2-(4-(3-Trifluoromethyl-phenyl)piperazin-1-yl)ethyl]-2,3-dihydro-1H-benzimidazol-2-on, gegebenenfalls in Form der pharmazeutisch verträglichen Säureadditionssalze sowie gegebenenfalls in Form der Hydrate oder Solvate zur Herstellung eines Arzneimittels zur Behandlung und/oder Vorbeugung von neurodegenerativen Erkrankungen sowie Gehimischämie verschiedener Genese ausgewählt aus der Gruppe bestehend aus Status epileptikus, Hypoglykämie, Hypoxie, Anoxie, Gehimtrauma, Gehimoedem, amyotrope laterale Sklerose, Morbus Alzheimer, Hypotonie, Herzinfarkt, Himdruck (erhöhter intrakranialer Druck), ischämischer und hämorrhagischer Stroke (Schlaganfall), globale cerebrale Ischämie bei Herzstillstand, Diabetische Polyneuropathie, Tinitus, perinatale Asphyxie, Herzhypertrophie (Herzmuskelverdickung), Herzinsuffizienz (Herzmuskelschwäche) und Morbus Parkinson.

Besonders bevorzugt betrifft die vorliegende Erfindung die Verwendung von 1-[2-(4-(3-Trifluoromethyl-phenyl)piperazin-1-yl)ethyl]-2,3-dihydro-1 H-benzimidazol-2-on, gegebenenfalls in Form der pharmazeutisch verträglichen Säureadditionssalze sowie gegebenenfalls in Form der Hydrate oder Solvate zur Herstellung eines Arzneimittels zur Behandlung und/oder Vorbeugung von Erkrankungen ausgewählt aus der. Gruppe bestehend aus Himdruck (erhöhter intrakranialer Druck), ischämischer und hämorrhagischer Stroke (Schlaganfall), Herzhypertrophie (Herzmuskelverdickung) und Herzinsuffizienz (Herzmuskelschwäche), wobei der erfindungsgemäßen Verwendung zur Behandlung und/oder Vorbeugung des Schlaganfalls besondere Bedeutung zukommt.

Gegebenenfalls kann die erfindungsgemäße Verwendung von 1-[2-(4-(3-Trifluoromethyl-phenyl)piperazin-1-yl)ethyl]-2,3-dihydro-1 H-benzimidazol-2-on in Kombination mit anderen Wirkstoffen erfolgen. Diese Wirkstoffe sind beispielsweise ausgewählt aus der Gruppe der Glutamat-Rezeptorantagonisten, der Calciumkanalblocker, der Natriumkanalblocker, der pharmazeutisch verträglichen Radikalfänger, der 5HT_{1A}-agonisten, der Endothelinantagonisten oder auch der Proteasome-inihibtoren. Als mögliche Kombinationspartner im Rahmen der erfindungsgemäßen Verwendung von 1-[2-(4-(3-Trifluoromethyl-phenyl)piperazin-1-yl)ethyl]-2,3-dihydro-1 H-benzimidazol-2-on seien bevorzugterweise genannt Magnesiumsalze, bevorzugt Magnesiumsulfat, Neramexane, Zonampenel, NS 1209, UK-315716, Sipatrigine, Crobenetine, Irampanel, NS-7, Harmokisane, Radicut, CPI-22, DY-9760, Repinotan, SUN-N4057, S-0139, Citicoline oder auch MLN-519. Als besonders bevorzugte Kombinationspartner im Rahmen der erfindungsgemäßen Verwendung von 1-[2-(4-(3-Trifluoromethyl-phenyl)piperazin-1-yl)ethyl]-2,3-dihydro-1H-benzimidazol-2-on seien genannt Magnesiumsulfat, Sipatrigine, Crobenetine, Irampanel und NS-7.

Unter pharmazeutisch verträglichen Säureadditionssalzen werden erfindungsgemäß solche Salze verstanden, die ausgewählt sind aus den Salzen der Salzsäure, Bromwasserstoffsäure, Schwefelsäure, Phosphorsäure, Methansulfonsäure, Essigsäure, Fumarsäure, Bernsteinsäure, Milchsäure, Zitronensäure, Weinsäure und Maleinsäure, wobei die Salze der Salzsäure, Bromwasserstoffsäure, Schwefelsäure, Phosphorsäure, und Essigsäure besonders bevorzugt sind. Eine besondere Bedeutung kommt hierbei den Salzen der Salzsäure zu.

Alternativ zur Verwendung in Form ihrer vorstehend genannten pharmazeutisch verträglichen Säureadditionssalze kann die Verbindung 1-[2-(4-(3-Trifluoromethylphenyl)piperazin-1-yl)ethyl]-2,3-dihydro-1 H-benzimidazol-2-on auch in Form ihrer freien Base zur erfindungsgemäßen Verwendung eingesetzt werden. Die freie Base des 1-[2-(4-(3-Trifluoromethyl-phenyl)piperazin-1-yl)ethyl]-2,3-dihydro-1 H-benzimidazol-2-ons ist im Stand der Technik noch nicht bekannt. Es wurde gefunden, daß die freie Base des 1-[2-(4-(3-Trifluoromethyl-phenyl)piperazin-1-yl)ethyl]-2,3-dihydro-1 H-benzimidazol-2-ons in zwei unterschiedlichen Kristallmodifikationen, den Polymorphen A und B erhalten werden kann.

Die Entstehung der unterschiedlichen Polymorphe A und B ist entscheidend von der Wahl der bei der Herstellung zur Anwendung gelangenden Reaktionsbedingungen abhängig. Im Rahmen der vorliegenden Erfindung kommt der Verwendung des Polymorphs A der freien Base des 1-[2-(4-(3-Trifluoromethyl-phenyl)piperazin-1-yl)ethyl]-2,3-dihydro-1 H-benzimidazol-2-ons zur Herstellung eines Arzneimittels mit neuroprotektiver Wirkung eine besondere Bedeutung zu.

Polymorph A des 1-[2-(4-(3-Trifluoromethyl-phenyl)piperazin-1-yl)ethyl]-2,3-dihydro-1H-benzimidazol-2-ons ist gekennzeichnet durch einen Schmelzpunkt von 161 °C (bestimmt über DSC; Heizrate 10 K/min). Polymorph B weist einen Schmelzpunkt von 120°C (bestimmt über DSC; Heizrate 10 K/min) auf. DSC steht für Differential Scanning Calorimetry.

Ein möglicher synthetischer Zugang zur Herstellung der freien Base des 1-[2-(4-(3-Trifluoromethyl-phenyl)piperazin-1-yl)ethyl]-2,3-dihydro-1H-benzimidazol-2-ons, insbesondere zur Herstellung des Polymorps A, ist der nachfolgenden, beispielhaft erläuterten experimentellen Vorgehensweise zu entnehmen.

### Beispiel:

375 kg 1-[(3-Trifluoromethyl)phenyl]-4-(2-cloroethyl)piperazin werden in einem geeigneten Raktor in 2500 kg Wasser aufgenommen und mit 200 kg wässeriger NaOH-Lösung (45%-ig) versetzt. Unter Rühren werden 169.2 kg 1-(2-Propenyl)-1,3-dihydro-benzimidazol-2H-on, 780 kg Isopropanol, 2000 kg Wasser und 220 kg wässerige NaOH-Lösung (45%-ig) zugesetzt. Die Reaktionsmischung wird auf 75-85°C erhitzt und nacheinander mit 160 kg konzentrierter Salzsäure und 200 kg Wasser versetzt. Die erhaltene Mischung wird für etwa 45 Minuten bei konstanter Temperatur gerührt. Nach Abdestillieren einer Mischung von Wasser und Isopropanol (ungefähr 3000 kg) wird der verbleibende Rückstand auf etwa 65-75°C gekühlt und ein pH-Wert von etwa 6.5 - 7.5 mittels 125 kg kg wässeriger NaOH-Lösung (45%-ig) eingestellt. Nach Abkühlen auf 45-50°C wird der pH durch Zugabe von 4 kg wässeriger NaOH-Lösung (45%-ig) auf etwa 8-9 eingestellt. Anschließend wird die erhaltene Mischung auf 30-35°C abgekühlt und zentrifugiert. Der so erhaltene Rückstand wird mit 340 I Wasser und 126 I Isopropanol gewaschen. Das erhaltene Produkt wird im Vakuum bei etwa 45-55°C getrocknet. Ausbeute: 358 kg Rohprodukt;
Das Rohprodukt wird in einem geeigneten Raktor in 1750 kg Aceton aufgenommen und die erhaltene Mischung unter Rühren bis zum Rückfluß erhitzt. Die erhaltene Lösung wird filtriert, das Filtrat anschließend mittels Destillation konzentriert. Anschließend wird für etwa 1 Stunde auf 0-5°C abgekühlt, der auskristallisierte Feststoff abfiltriert und abschließend bei etwa 55°C für etwa 12 Stunden getrocknet. Ausbeute: 280 kg Polymorph A.

Geeignete pharmazeutische Anwendungsformen von 1-[2-(4-(3-Trifluoromethylphenyl)piperazin-1-yl)ethyl]-2,3-dihydro-1 H-benzimidazol-2-on im Sinne der erfindungsgemäßen Verwendung sind beispielsweise Tabletten, Kapseln, Zäpfchen, Lösungen, - insbesondere Lösungen zur Injektion (s.c., i.v., i.m.) und Infusion - Säfte, Emulsionen oder dispersible Pulver. Hierbei soll der Anteil der pharmazeutisch wirksamen Verbindung jeweils im Bereich von 0,1 bis 90 Gew.-%, bevorzugt 0,5 bis 50 Gew.-% der Gesamtzusammensetzung liegen, d.h. in Mengen die ausreichend sind, um den unten angegebenen Dosierungsbereich zu erreichen.

Die Dosierung von Flibanserin in der erfindungsgemäßen Verwendung kann beispielsweise in einem Bereich von etwa 0,1 - 500 mg Flibanserin pro Tag liegen. Bevorzugt liegt die Dosierung in einem Bereich von etwa 1 - 300 mg/Tag, besonders bevorzugt in einem Bereich von etwa 2-200mg/Tag bezogen auf Flibanserin in Form seiner freien Base. Für den Fachmann ist natürlich ersichtlich, daß es gegebenenfalls erforderlich sein kann, von den genannten Mengen abzuweichen, und zwar in Abhängigkeit vom Körpergewicht bzw. der Art des Applikationsweges, vom individuellen Verhalten gegenüber dem Medikament, der Art von dessen Formulierung und dem Zeitpunkt bzw. Intervall, zu welchen die Verabreichung erfolgt. So kann es in einigen Fällen ausreichend sein, mit weniger als der vorgenannten Mindestmenge auszukommen, während in anderen Fällen die genannte obere Grenze überschritten werden muß. Im Falle der Applikation größerer Mengen kann es empfehlenswert sein, diese in mehreren Einzelgaben über der Tag zu verteilen.

Den Wirkstoff enthaltende Tabletten können beispielsweise durch Mischen des oder der Wirkstoffe mit bekannten Hilfsstoffen, beispielsweise inerten Verdünnungsmitteln, wie Calciumcarbonat, Calciumphosphat oder Milchzucker, Sprengmitteln, wie Maisstärke oder Alginsäure, Bindemitteln, wie Stärke oder Gelatine, Schmiermitteln, wie Magnesiumstearat oder Talk, und/oder Mitteln zur Erzielung des Depoteffektes, wie Carboxymethylcellulose, Celluloseacetatphthalat, oder Polyvinylacetat erhalten werden. Die Tabletten können auch aus mehreren. Schichten bestehen.

Entsprechend können Dragees durch Überziehen von analog den Tabletten hergestellten Kernen mit üblicherweise in Drageeüberzügen verwendeten Mitteln, beispielsweise Kollidon oder Schellack, Gummi arabicum, Talk, Titandioxid oder Zucker, hergestellt werden. Zur Erzielung eines Depoteffektes oder zur Vermeidung von Inkompatibilitäten kann der Kern auch aus mehreren Schichten bestehen. Desgleichen kann auch die Drageehülle zur Erzielung eines Depoteffektes aus mehreren Schichten bestehen wobei die oben bei den Tabletten erwähnten Hilfsstoffe verwendet werden können.

Säfte der erfindungsgemäßen Wirkstoffe beziehungsweise Wirkstoffkombinationen können zusätzlich noch ein Süßungsmittel, wie Saccharin, Cyclamat, Glycerin oder Zucker sowie ein geschmacksverbesserndes Mittel, z.B. Aromastoffe, wie Vanillin oder Orangenextrakt, enthalten. Sie können außerdem Suspendierhilfsstoffe oder Dickungsmittel, wie Natriumcarboxymethylcellulose, Netzmittel, beispielsweise Kondensationsprodukte von Fettalkoholen mit Ethylenoxid, oder Schutzstoffe, wie p-Hydroxybenzoate, enthalten.

Injektions- und Infusionslösungen werden in üblicher Weise, z.B. unter Zusatz von Isotonantien, Konservierungsmitteln, wie p-Hydroxybenzoaten, oder Stabilisatoren, wie Alkalisalzen der Ethylendiamintetraessigsäure, gegebenenfalls unter Verwendung von Emulgiermitteln und /oder Dispergiermitteln, wobei beispielsweise bei der Verwendung von Wasser als Verdünnungsmittel gegebenenfalls organische Lösemittel als Lösevermittler bzw. Hilflösemittel eingesetzt werden können, hergestellt und in Injektionsflaschen oder Ampullen oder Infusionsflaschen abgefüllt.

Die eine oder mehrere Wirkstoffe beziehungsweise Wirkstoffkombinationen enthaltenden Kapseln können beispielsweise hergestellt werden, indem man die Wirkstoffe mit inerten Trägern, wie Milchzucker oder Sorbit, mischt und in Gelatinekapseln einkapselt.
Geeignete Zäpfchen lassen sich beispielsweise durch Vermischen mit dafür vorgesehenen Trägermitteln, wie Neutralfetten oder Polyäthylenglykol beziehungsweise dessen Derivaten, herstellen.
Als Hilfsstoffe seien beispielsweise Wasser, pharmazeutisch unbedenkliche organische Lösemittel, wie Paraffine (z.B. Erdölfraktionen), Öle pflanzlichen Ursprungs (z.B. Erdnuß- oder Sesamöl), mono- oder polyfunktionelle Alkohole (z.B. Ethanol oder Glycerin), Trägerstoffe wie z.B. natürliche Gesteinsmehle (z.B. Kaoline, Tonerden, Talkum, Kreide) synthetische Gesteinsmehle (z.B. hochdisperse Kieselsäure und Silikate), Zucker (z.B. Rohr-, Milch- und Traubenzucker) Emulgiermittel (z.B. Lignin, Sufitablaugen, Methylcellulose, Stärke und Polyvinylpyrrolidon) und Gleitmittel (z.B. Magnesiumstearat, Talkum, Stearinsäure und Natriumlaurylsulfat) erwähnt.

Die Applikation erfolgt in üblicher Weise, vorzugsweise parenteral - insbesondere auf dem Wege der Infusion - intravenös. Im Falle der oralen Anwendung können die Tabletten selbstverständlich außer den genannten Trägerstoffen auch Zusätze, wie z.B. Natriumcitrat, Calciumcarbonat und Dicalciumphosphat zusammen mit verschiedenen Zuschlagstoffen, wie Stärke, vorzugsweise Kartoffelstärke, Gelatine und dergleichen enthalten. Weiterhin können Gleitmittel, wie Magnesiumstearat, Natriumlaurylsulfat und Talkum zum Tablettieren mitverwendet werden. Im Falle wäßriger Suspensionen können die Wirkstoffe außer den obengenannten Hilfsstoffen mit verschiedenen Geschmacksaufbesserem oder Farbstoffen versetzt werden. Für den Fall der parenteralen Anwendung können Lösungen der Wirkstoffe unter Verwendung geeigneter flüssiger Trägermaterialien eingesetzt werden. Eine parenterale Applikation kann beispielsweise durch Infusionen erfolgen, die je nach Krankheitsbild unter Umständen auch über einen längeren Zeitraum (Stunden oder Tage) zur Anwendung gelangen können. Die nachfolgenden Formulierungsbeispiele, die nach gängigen Verfahren herstellbar sind, illustrieren die vorliegende Erfindung ohne sie jedoch in ihrem Umfang zu beschränken:

### Pharmazeutische Formulierungsbeispiele

| A) | Tabletten | pro Tablette |
|---|---|---|
| | Flibanserin x HCl | 10 mg |
| | Lactose | 187 mg |
| | Maisstärke | 50 mg |
| | Magnesiumstearat | 3 mg |
| | | 250 mg |

| B.) | Tabletten | pro Tablette |
|---|---|---|
| | Flibanserin (freie Base) | 80 mg |
| | Lactose | 88 mg |
| | Maisstärke | 190 mg |
| | Mikrokristalline Cellulose | 40 mg |
| | Magnesiumstearat | 2 mg |
| | | 400 mg |

| C) | Kapseln | pro Tablette |
|---|---|---|
| | Flibanserin (freie Base) | 10 mg |
| | Lactose | 188 mg |
| | Magnesiumstearat | 2 mg |
| | | 200 mg |

Vorstehende Mischung kann in geeignete Hartgelatinekapseln abgefüllt werden.

| | | |
|---|---|---|
| D) | Ampullenlösung | |
| | Flibanserin x HCl | 2 mg |
| | Natriumchlorid | 9 mg |
| | Aqua pro inj. 5 ml | |

## Patentansprüche

1. Verwendung von 1-[2-(4-(3-Trifluoromethyl-phenyl)piperazin-1-yl)ethyl]-2,3-dihydro-1H-benzimidazol-2-on, gegebenenfalls in Form der pharmazeutisch verträglichen Säureadditionssalze sowie gegebenenfalls in Form der Hydrate oder Solvate zur Herstellung eines Arzneimittels zur Behandlung und/oder Vorbeugung von neurodegenerativen Erkrankungen sowie Gehirnischämie verschiedener Genese.

2. Verwendung nach Anspruch 1, zur Behandlung und/oder Vorbeugung von Erkrankungen ausgewählt aus der Gruppe bestehend aus Status epileptikus, Hypoglykämie, Hypoxie, Anoxie, Gehimtrauma, Gehirnoedem, amyotrope laterale Sklerose, Morbus Alzheimer, Hypotonie, Herzinfarkt, Himdruck (erhöhter intrakranialer Druck), ischämischer und hämorrhagischer Stroke (Schlaganfall), globale cerebrale Ischämie bei Herzstillstand, Diabetische Polyneuropathie, Tinitus, perinatale Asphyxie, Herzhypertrophie (Herzmuskelverdicküng), Herzinsuffizienz (Herzmuskelschwäche) und Morbus Parkinson.

3. Verwendung nach Anspruch 2 zur Behandlung und/oder Vorbeugung von Erkrankungen ausgewählt aus der Gruppe bestehend aus Himdruck (erhöhter intrakranialer Druck), ischämischer und hämorrhagischer Stroke (Schlaganfall), Herzhypertrophie (Herzmusketverdickung) und Herzinsuffizienz (Herzmuskelschwäche).

4. Verwendung nach einem der Ansprüche 1 bis 3, **dadurch gekennzeichnet, daß** 1-[2-(4-(3-Trifluoromethyl-phenyl)piperazin-1-yl)ethyl]-2,3-dihydro-1 H-benzimidazol-2-on in Form eines seiner pharmazeutisch verträglichen Säureadditionssalzen eingesetzt wird, die ausgewählt sind aus der Gruppe bestehend aus den Salzen der Salzsäure, Bromwasserstoffsäure, Schwefelsäure, Phosphorsäure, Methansulfonsäure, Essigsäure, Fumarsäure, Bernsteinsäure, Milchsäure, Zitronensäure, Weinsäure und Maleinsäure.

5. Verwendung nach einem der Ansprüche 1 bis 3, **dadurch gekennzeichnet, daß** 1-[2-(4-(3-Trifluoromethyl-phenyl)piperazin-1-yl)ethyl]-2,3-dihydro-1H-benzimidazol-2-on in Form seiner freien Base verwendet wird.

6. Verwendung nach Anspruch 5, **dadurch gekennzeichnet, daß** 1-[2-(4-(3-Trifluoromethyl-phenyl)piperazin-1-yl)ethyl]-2,3-dihydro-1H-benzimid azol-2-on in Form des Polymorphs A der freien Base, welches durch einen Schmelzpunkt von etwa 161°C (bestimmt über DSC; Heizrate 10 K/min) **gekennzeichnet** ist, verwendet wird.

## Claims

1. Use of 1-[2-(4-(3-trifluoromethyl-phenyl)piperazin-1-yl)ethyl]-2,3-dihydro-1H-benzimidazol-2-one, optionally in the form of the pharmaceutically acceptable acid addition salts and optionally in the form of the hydrates or solvates, for preparing a pharmaceutical composition for the treatment and/or prevention of neurodegenerative diseases as well as cerebral ischaemia of various origins.

2. Use according to claim 1, for the treatment and/or prevention of diseases selected from the group consisting of epilepsy, hypoglycaemia, hypoxia, anoxia, brain trauma, brain oedema, amyotropic lateral sclerosis, Alzheimer's disease, hypotension, cardiac infarct, brain pressure (elevated intracranial pressure), ischaemic and haemorrhagic stroke (stroke), global cerebral ischaemia during stoppage of the heart, diabetic polyneuropathy, tinnitus, perinatal asphyxia, cardiac hypertrophia (thickening of the heart muscle), cardiac insufficiency (weakness of the heart muscle) and Parkinson's disease.

3. Use according to claim 2 for the treatment and/or prevention of diseases selected from the group consisting of brain pressure (elevated intracranial pressure), ischaemic and haemorrhagic stroke (stroke), cardiac hypertrophia (thickening of the heart muscle) and cardiac insufficiency (weakness of the heart muscle).

4. Use according to one of claims 1 to 3, **characterised in that** 1-[2-(4-(3-trifluoromethyl-phenyl)piperazin-1-yl)ethyl]-2,3-dihydro- 1H-benzimidazol-2-one is used in the form of one of the pharmaceutically acceptable acid addition salts thereof, which are selected from the group consisting of the salts of hydrochloric acid, hydrobromic acid, sulphuric acid, phosphoric acid, methanesulphonic acid, acetic acid, fumaric acid, succinic acid, lactic acid, citric acid, tartaric acid and maleic acid.

5. Use according to one of claims 1 to 3, **characterised in that** 1-[2-(4-(3-trifluoromethyl-phenyl)piperazin-1-yl) ethyl] -2,3-dihydro-1 1H-benzimidazol-2-one is used in the form of its free base.

6. Use according to claim 5, **characterised in that** 1-[2-(4-(3-trifluoromethyl-phenyl)piperazin-1-yl)ethyl]-2,3-dihydro-1H-benzimidazol-2-one is used in the form of polymorph A of the free base, which is **characterised by** a melting point of about 161°C (measured by DSC; heating rate 10 K/min).

## Revendications

1. Utilisation de la 1-[2-(4-(3-trifluorométhyl-phényl)pipérazin-1-yl)-éthyl]-2,3-dihydro-1H-benzimidazol-2-one, éventuellement sous forme des sels d'addition d'acide pharmaceutiquement acceptables ainsi que éventuellement sous forme des hydrates ou des solvates pour la production d'un médicament pour le traitement et/ou la prévention des maladies neurodégénératives ainsi que de l'ischémie cérébrale de genèse diverse.

2. Utilisation selon la revendication 1 pour le traitement et/ou la prévention de maladies choisies dans le groupe consistant en l'épilepsie, l'hypoglycémie, l'hypoxie, l'anoxie, les traumatismes cérébraux, l'oedème cérébral, la sclérose latérale amyotropique, la maladie d'Alzheimer, l'hypotonie, l'infarctus cardiaque, la pression cérébrale (pression intra-crânienne augmentée), l'attaque ischémique et hémorragique (apoplexie cérébrale), l'ischémie cérébrale globale au repos cardiaque, la polyneuropathie diabétique, l'acouphène, l'asphyxie périnatale, l'hypertrophie cardiaque (épaississement du muscle cardiaque), l'insuffisance cardiaque (faiblesse du muscle cardiaque) et la maladie de Parkinson.

3. Utilisation selon la revendication 2 pour le traitement et/ou la prévention de maladies choisies dans le groupe consistant en la pression cérébrale (pression intra-crânienne augmentée), l'attaque ischémique et hémorragique (apoplexie cérébrale), l'hypertrophie cardiaque (épaississement du muscle cardiaque) et l'insuffisance cardiaque (faiblesse du muscle cardiaque).

4. Utilisation selon l'une des revendications 1 à 3 **caractérisée en ce que** la 1-[2-(4-(3-trifluorométhyl-phényl)pipérazin-1-yl)éthyl]-2,3-dihydro-1H-benzimidazol-2-one est utilisée sous forme de l'un de ses sels d'addition d'acide pharmaceutiquement acceptables qui sont choisis dans le groupe consistant en les sels de l'acide chlorhydrique, de l'acide bromhydrique, de l'acide sulfurique, de l'acide phosphorique, de l'acide méthanesulfonique, de l'acide acétique, de l'acide fumarique, de l'acide succinique, de l'acide lactique, de l'acide citrique, de l'acide tartrique et de l'acide maléique.

5. Utilisation selon l'une des revendications 1 à 3 **caractérisée en ce que** la 1-[2-(4-(3-trifluorométhyl-phényl)pipérazin-1-yl)éthyl]-2,3-dihydro-1H-benzimidazol-2-one est utilisée sous forme de sa base libre.

6. Utilisation selon la revendication 5 **caractérisée en ce que** la 1-[2-(4-(3-trlfluorométhyl-phényl)pipérazin-1-yl)éthyl]-2,3-dihydro-1H-benzimidazol-2-one est utilisée sous forme du polymorphe A de la base libre, qui est **caractérisé par** un point de fusion d'environ 161°C (déterminé par DSC ; vitesse de chauffage 10 K/min).
